# EUROPEAN PATENT SPECIFICATION

(11) **EP 1 091 001 B1**
(45) Date of publication and mention of the grant of the patent: **05.10.2005**
(21) Application number: 00120521.0
(22) Date of filing: 20.09.2000
(51) Int. Cl.: C12P 19/40, C07H 19/16

(54) **Process for the purification of S-adenosyl-L-methionine and for the preparation of the pharmaceutically acceptable salts thereof**
Verfahren zur Reinigung von S-Adenosyl-L-Methionin und zur Herstellung von pharmazeutisch verträglichen Salzen davon
Procédé de purification de S-adénosyl-L-méthionine et pour la préparation des sels pharmaceutiquement acceptables

(30) Priority: 05.10.1999 IT MI992070
(43) Date of publication of application: 11.04.2001
(73) Proprietor: CHEMENTECNO S.r.l., 20052 Monza, (MI) (IT); GNOSIS SRL, 21050 Cairate (Varese) (IT)
(72) Inventor: Polastri, Franco, 20052 Monza (MI) (IT); Santambrogio, Gianni, 20052 Monza (MI) (IT); Sivieri, Lino, 20052 Monza (MI) (IT)
(74) Representative: Mancini, Vincenzo

(56) References cited:
- EP-A- 0 141 914
- DE-A- 1 803 978
- US-A- 3 962 034
- US-A- 4 028 183
- US-A- 4 562 149

## Description

The invention relates to a process for the purification of S-adenosyl-L-methionine (hereinafter SAMe) and for the preparation of the pharmaceutically acceptable salts thereof. Particularly, the invention relates to a process for the purification of SAMe and for the preparation of the pharmaceutically acceptable salts thereof starting from yeasts enriched with SAMe.

As it is well known, SAMe is a physiological methyl donor involved in enzymatic transmethylation reactions, which can be found in any living organism, and having therapeutical effects towards chronic hepatic pathologies, adiposis, lipemia, arteriosclerosis, and is therefore desirable to produce it in great quantities.

Yet, an obstacle to the use of the molecule of interest, on an industrial scale, has been observed because of its thermal instability, even at room temperature, and of the complexity of the processes for its preparation and purification.

Several processes for the purification of SAMe and for the preparation of its pharmaceutically acceptable salts are known.

Yet, the known processes of purification comprise the use of strong acid resins (JP 13680/1971) or chelated resins (JP 20998/1978) or they use peculiar expensive reactants such as picric and picrolinic acids (US 3707536 and US 3954726).

Processes of purification using weak acid resins are also known (JP 14299/1981, FR-A-2531714, EP-A-0141914) yet, they allow only a partial separation of SAMe and therefore an insufficient purity which does not permit the pharmaceutical use of the product.

However, even if carrying out some of the above mentioned processes allows to obtain a higher purity, in some occurrences (FR-2531714), the use of potassium bicarbonate is contemplated in order to extract the product from cells, obtaining the precipitation of potassium perchlorate as a consequence, which entails problems relating separation first, and then waste. To obtain a good purity grade, in the occurrence of EP-A-0141914, the use of chromatographic columns comprising 100-200 mesh resins is provided indeed, entailing high costs insofar as investment and service are both concerned.

As to the pharmaceutically acceptable salts of SAMe, the known processes (US 3893999, US 3954726, US 4057686, EP-A-0073376) relate mainly to the obtainment of specific salts and result to be, however, unsuitable on an industrial scale, also because of the use of expensive reactants and of the huge reaction volumes required.

Also EP-A-0141914, regardless of the fact it describes a process allowing the production of stable SAMe salts, applicable on an industrial scale and with better yields in comparison with what can be obtained by the processes above scrutinised, entails the enrichment of the yeast containing SAMe and the subsequent lysis of the cells in the presence of an organic solvent (for instance ethyl acetate, acetone, etc.) further using, as it has already been mentioned, huge amounts of a resin having a particle size of 100-200 mesh. The use of solvents for the extraction of SAMe necessarily entails employing antidetonative plants, recovery and distillation systems and solvent recovery system, besides to the needed drying of the exhausted mycelium, in order to avoid to discharge it with some residual solvent, which plainly entails further investments and production costs.

According to a first aspect the present invention concerns a process for the purification of SAMe from enriched yeast, containing at least 10 g/l of SAMe, comprising:
(a) - adjusting pH to 1.8-2.2;
(b) - preparing an aqueous lysate of SAMe from the enriched yeast, at a temperature equal to 70-92°C, letting the yeast pass through a plate-type exchanger, in countercurrent, for a contact time equal to 5-45 seconds;
(c) - cooling the lysate at a temperature equal to 2-30°C;
(d) - ultrafiltering the lysate and the subsequent adjusting of pH to 3.0-6.8;
(e) - adsorbing at least 90 g/l of the ultrafiltrate on a weak acid resin, where the particle size are equal to 30-50 mesh, eluting with a 0.1-2N solution of an inorganic acid;
(f) - adjusting pH to 2.0-2.5;
(g) - decolorizing the eluate;
(h) - concentrating the eluate;
(i) - freeze-drying the eluate.

According to a preferred aspect, temperature in step (c) is equal to 6-8°C, whereas pH of step (d) is adjusted to 4.8-5.2.

The ultrafiltrate deriving from step (d) is preferably adsorbed on the resin until its saturation.

The process of the invention allows to avoid the use of organic solvents in the preparation of the lysate with plain advantages insofar as the steps relating to the purification of SAMe and/or its pharmaceutically acceptable salts, as well as ecology and environment, are concerned.

The present invention further allows the use of reduced amounts of resin, having also common particle size, equal to 30-50 mesh, and therefore results to be economically advantageous.

It is further possible to obtain the pharmaceutically acceptable salts of SAMe having yield and purity higher than the ones obtainable by the known processes; a grade at least equal to 98% of SAMe and a yield equal to at least 90% based on the fermentation product are indeed obtained.

Because of its peculiar conditions, the process of the invention allows to avoid the degradation of SAMe during the preparation of the lysate, succeeding in obtaining a lysis with a yield higher than 98% and a content of side products, the main of which is 5-deacyl-5-methylthioadenosine, lower than 1%.

Adversely to what disclosed in the cited prior art (EP-0141914), it has been further observed that the absorption of huge amounts, at least equal to 90 g/l of SAMe, and preferably till saturation of the resin is attained, instead of the common 5-30 g/l, where the resin shows a particle size equal to 30-50 mesh instead of 100-200 mesh, entails a remarkable lowering of costs and the reduction of the charge loss on an industrial scale.

Such surprising conditions relating to the charge of the resin entail, as already mentioned, a basically full purification and, at the same time, allow to drastically lower the required amounts of the resin.

The direct release of impurities during the absorption of at least 90 g/l on the resin has also plainly resulted and, as a consequence, the uselessness of carrying out further elutions, adversely to what provided in the above mentioned prior art in the intent of directly obtain a high quantity of the final product.

For instance, SAMe can be produced by fermentating a suitable micro-organism such as *Saccharomyces pastorianus* (formerly known as *Saccharomyces carlsbergensis* CBS 1513), *Saccharomyces cerevisiae* (IFO 2044), *Torulopsis utilis* and *Candida utilis*.

The yeast containing SAMe can be enriched by the processes known in the art such as, for instance, the Schlenk method disclosed in "The Journal of Biological Chemistry", vol. 29, pag. 1037, (1987).

The SAMe enriched yeast, containing approximately at least 10 g/l of SAMe, in order to be advantageously used in carrying out the present invention, is subjected to a process of cell lysis, after having adjusted the pH to 1.8-2.2, letting the yeast pass through a double-type pressure exchanger, at a temperature equal to 70-92°C and for a contact time equal to 5-45 seconds.

After having been cooled at a temperature equal to 2-30°C, the resulting lysate is subjected to ultrafiltration, subsequently adjusting the pH to 3.0-6.8.

The ultrafiltrate is then absorbed on a weak acid resin, preferably a cationic, carboxylic one, such as Rohm and Haas® IRC86, having a particle size equal to 30-50 mesh, till at least 90 g/l, preferably till saturation, finally eluting with a solution of an inorganic acid such as, for instance, 0.1-2N sulphuric, hydrochloric, nitric acid, etc.

After adjusting the pH to 2.0-2.5, the decolorization of the eluate is carried out, for instance by a copolymeric styrene-divinylbenzene resin, such as Resindion® SP 825L, or using molecular sieves, subsequently carrying out the concentration and finally the freeze-drying of the resulting solution.

The SAMe so obtained shows a titre higher than 98% as an average.

According to another aspect, the present invention discloses a process, for the preparation of the pharmaceutically acceptable salts of SAMe, which comprises the purification of SAMe according to what has already been described and wherein, after decolorizing the eluate in step (g), stoicheiometric amounts of pharmaceutically acceptable acids, even in admixture among them, preferably sulphuric and paratoluenesolfonic acids, are added to said eluate, in order to obtain the corresponding pharmaceutically acceptable salts of SAMe.

The following examples illustrate the invention without limiting.

### EXAMPLE 1

1000 l of yeast obtained by fermentation of *Saccharomyces carlsbergensis*, enriched in SAMe according to the Schlenk method (average titre 10 g/l), adjusted to pH 2 with H₂SO₄, were passed through two plate exchangers (produced by ALFA-LAVAL; plates: 0,5 m² + 3 m²) by a metering pump (produced by CODIP) at the temperature of 76°C-78°C, with a solution of hot water at 88°C. From room temperature to 76°C-78°C, the heating time was about 8 seconds, letting such a temperature for 20-45 seconds. The solution was then cooled first by using cool water and then brine, till the solution was brought to a temperature of about 6°C.

The solution was passed through an ultrafiltration membrane HYDRO AIR to discard the solid consisting of mycelium residuals.

The pH of the solution was finally brought at 4.8-5.2 by adding 28% NH₄OH.

Column absorption on a IRC 86 resin (Rohm and Haas® - 30-50 mesh) was carried out with a flow equal to 0.8-2 BV/h (liquid amount passing through the resin/hour) with respect to 1000 l of resin.

The absorption has to be carried out till at lest 90 g/l are charged on the column. From 60 g/l on, given the selectivity of the type of resin, impurities present in the absorbed solutions, and particularly adenosine, are directly discarded during absorption.

Eluting with 0.5N H₂SO₄, discarding the first fractions containing impurities, was then carried out.

The collected eluate was adjusted to pH 2-2.5 by NH₄OH, then passed through a molecular sieve for decolorizing (and eluting possible traces of methylthioadenosine). 93 KA (kiloactivity) of SAMe, to which the corresponding amounts of sulphuric acid and paratoluenesolfonic acid were added, were obtained.

The solution was then piped to freeze-drying; as an average, 170-175 kg of SAMe sulphate paratoluensolfonate with a titre ≥98%, were obtained.

### EXAMPLE 2

500 l of yeast obtained by fermentation of *Saccharomyces carlsbergensis* enriched in SAMe according to the method described in example 1, having an activity equal to 12 g/l, were brought to a temperature of about 5°C at a pH ≈2 by adding sulphuric acid and then passed through two plate exchangers produced by ALFA-LAVAL (0,25 m² and 1,5 m², respectively) and maintained at 78°C for 25 seconds.

The solution was cooled with cool water till 6-8°C at the outlet of the exchanger.

The solution, after having been passed through an ultrafiltration membrane HYDRO AIR, in order to discard the suspended solids, was filtered, always maintaining temperature at about 6-8°C, and adjusting the pH to 4.8-5.2 by adding 28% NH₄OH.

The composition of impurities is by now of ≈0,6% of adenosine and of ≈0,5% of methylthioadenosine.

The absorption on a column containing 700 l of an IRC 86 resin, Rohm and Haas® (30-50 mesh), was then carried out.

The absorption was carried out till all the solution was charged: as the resin came to saturation with SAMe, it released the adenosine previously hold which was discarded in the same absorption step.

The eluate (the elution was carried out with 1% H₂SO₄) was directly passed through a Resindion® SP 825L resin in order to decolorize it and then piped to freeze-drying, after having concentrated it by nanofiltration, corrected the H₂SO₄ content and added paratoluenesolfonic acid.

100-105 kg of SAMe disulphate paratoluenesolfonate, having a titre ≥98%, were obtained.

## Claims

1. A process for the purification of S-adenosyl-L-methionine from enriched yeast, containing at least 10 g/l of it, which comprises:
(a) - adjusting pH to 1.8-2.2;
(b) - preparing an aqueous lysate of S-adenosyl-L-methionine from the enriched yeast, at a temperature equal to 70-92°C, letting the yeast pass through a plate-type exchanger, in countercurrent, for a contact time equal to 5-45 seconds;
(c) - cooling the lysate at a temperature equal to 2-30°C;
(d) - ultrafiltering the lysate and the subsequent adjusting of pH to 3.0-6.8;
(e) - adsorbing at least 90 g/l of the ultrafiltrate on a weak acid resin, where the particle size are equal to 30-50 mesh, eluting with a 0.1-2N solution of an inorganic acid;
(f) - adjusting pH to 2.0-2.5;
(g) - decolorizing the eluate;
(h) - concentrating the eluate;
(i) - freeze-drying the eluate.

2. A process according to claim 1, wherein the temperature in step (c) is 6-8°C.

3. A process according to claim 1 or 2, wherein the pH in step (d) is 4.8-5.2.

4. A process according to any of the foregoing claims, wherein the ultrafiltrate in step (e) is absorbed on the resin till saturation.

5. A process for the preparation of pharmaceutically acceptable salts of S-adenosyl-L-methionine comprising a process according to any of claims 1-4, wherein after having decolorized the eluate in step (g) stoicheiometric amounts of pharmaceutically acceptable acids, even in admixture among them, are added to it, in order to obtain the corresponding pharmaceutically acceptable salts of S-adenosyl-L-methionine.

6. A process according to claim 5, wherein the pharmaceutically acceptable acid is selected from sulphuric acid and paratoluenesolfonic acid.

## Patentansprüche

1. Verfahren für die Reinigung von S-Adenosyl-L-methionin aus angereicherter Hefe, die wenigstens 10 g/l davon enthält, welches umfasst:
(a) - Einstellen des pH auf 1,8-2,2;
(b) - Herstellen eines wässrigen Lysats von S-Adenosyl-L-methionin von der angereicherten Hefe bei einer Temperatur von 70-92°C, Passieren lassen der Hefe durch einen Austauscher vom Plattentyp, in Gegenstrom, für eine Kontaktzeit von 5-45 Sekunden;
(c) - Abkühlen des Lysats bei einer Temperatur von 2-30°C;
(d) - Ultrafiltrieren des Lysats und das nachfolgende Einstellen des pHs auf 3,0-6,8;
(e) - Adsorbieren von wenigstens 90 g/l des Ultrafiltrats an einem schwach sauren Harz, wobei die Partikelgröße gleich 30-50 mesh ist, Eluieren mit einer 0,1-2N Lösung einer anorganischen Säure;
(f) - Einstellen des pHs auf 2,0-2,5;
(g) - Entfärben des Eluats;
(h) - Konzentrieren des Eluats;
(i) - Gefriertrocknen des Eluats.

2. Verfahren nach Anspruch 1, wobei die Temperatur in Schritt (c) 6-8°C ist.

3. Verfahren nach Anspruch 1 oder 2, wobei der pH in Schritt (d) 4,8-5,2 ist.

4. Verfahren nach irgendeinem der vorangehenden Ansprüche, wobei das Ultrafiltrat in Schritt (e) an dem Harz bis zur Sättigung absorbiert wird.

5. Verfahren zur Herstellung pharmazeutisch annehmbarer Salze von S-Adenosyl-L-methionin umfassend ein Verfahren nach irgendeinem der Ansprüche 1-4, wobei, nachdem das Eluat in Schritt (g) entfärbt worden ist, ihm stöchiometrische Mengen pharmazeutisch annehmbarer Säuren, selbst in Mischung untereinander, zugesetzt werden, um die entsprechenden pharmazeutisch annehmbaren Salze von S-Adenosyl-L-methionin zu erhalten.

6. Verfahren nach Anspruch 5, wobei die pharmazeutisch annehmbare Säure ausgewählt ist aus Schwefelsäure und Paratoluolsolfonsäure.

## Revendications

1. Procédé pour la purification de *S*-adénosyl-*L*-méthionine à partir de levure enrichie, en contenant au moins 10 g/l, lequel comprend :
(a) - d'ajuster le pH à 1,8-2,2 ;
(b) - de préparer un lysat aqueux de *S*-adénosyl-*L*-méthionine à partir de la levure enrichie, à une température égale à 70-92°C, en permettant à la levure de passer à travers un échangeur de type échangeur à plateaux, à contre-courant, pendant un temps de contact égal à 5-45 secondes ;
(c) - de refroidir le lysat à une température égale à 2-30°C ;
(d) - d'effectuer une ultrafiltration du lysat et d'ajuster à la suite de cela le pH à 3,0-6,8 ;
(e) - d'adsorber au moins 90 g/l de l'ultrafiltrat sur une résine à acide faible, où la taille des particules est égale à 30-50 mesh, d'éluer avec une solution 0,1-2 N d'un acide inorganique ;
(f) - d'ajuster le pH à 2,0-2,5 ;
(g) - de décolorer l'éluat ;
(h) - de concentrer l'étuat ;
(i) - de lyophiliser l'éluat.

2. Procédé selon la revendication 1, dans lequel la température dans l'étape (c) est de 6-8°C.

3. Procédé selon la revendication 1 ou 2, dans lequel le pH dans l'étape (d) est de 4,8-5,2.

4. Procédé selon l'une quelconque des revendications précédentes, dans lequel l'ultrafiltrat dans l'étape (e) est absorbé sur la résine jusqu'à saturation.

5. Procédé pour la préparation de sels acceptables du point de vue pharmaceutique de *S*-adénosyl-*L*-méthionine comprenant un procédé selon l'une quelconque des revendications 1-4, dans lequel après avoir décoloré l'éluat dans l'étape (g) des quantités stoechiométriques d'acides acceptables du point de vue pharmaceutique, même de mélanges de ceux-ci, y sont ajoutées, afin d'obtenir les sels acceptables du point de vue pharmaceutique de *S*-adénosyl-*L*-méthionine correspondants.

6. Procédé selon la revendication 5, dans lequel l'acide acceptable du point de vue pharmaceutique est choisi entre l'acide sulfurique et l'acide *para*-toluènesulfonique.
